# EUROPEAN PATENT SPECIFICATION

(11) **EP 3 021 835 B1**
(45) Date of publication and mention of the grant of the patent: **10.05.2017**
(21) Application number: 14745327.8
(22) Date of filing: 16.07.2014
(51) Int. Cl.: A61K 9/08, A61K 47/02, A61K 47/38

(54) **COMPOSITION FOR APPLICATION TO A MUCOSA COMPRISING A HYDROXYALKYL METHYLCELLULOSE**
ZUSAMMENSETZUNG ZUR ANWENDUNG AUF EINER SCHLEIMHAUT MIT HYDROXYALKYL-METHYLCELLULOSE
COMPOSITION POUR APPLICATION À UNE MUQUEUSE COMPRENANT UN HYDROXY-ALKYLE MÉTHYLCELLULOSE

(30) Priority: 17.07.2013 US 201361847224 P
(43) Date of publication of application: 25.05.2016
(73) Proprietor: Dow Global Technologies LLC, Midland, MI 48674 (US)
(72) Inventor: CURTIS-FISK, Jaime L., Freeland, MI 48623 (US); JORDAN, Susan L., Doylestown, PA 18902 (US); ROGERS, True L., Midland, MI 48642 (US); APPELL, Robert B., Midland, MI 48640 (US); KNARR, Matthias, 31582 Nienburg/Weser (DE); ADDEN, Roland, 29664 Walsrode (DE)
(74) Representative: f & e patent
(86) International application number: PCT/US2014/046812
(87) International publication number: WO 2015/009799

(56) References cited:
- WO-A1-99/38492
- WO-A1-2012/051034
- WO-A1-2012/051035
- WO-A1-2013/154980
- WO-A1-2014/014752

## Description

### FIELD

The present invention concerns a composition for application to a mucosa, e.g. for transmucosal delivery of a physiologically active agent, and a method of administering a physiologically active agent to an individual.

### INTRODUCTION

Compositions for application to mucosae, such as pharmaceutical compositions for transmucosal delivery of physiologically active agents, have been known for a long time. Nasal drops and sprays have been known as drug delivery systems intended for administration to the nasal cavity.

WO99/38492 discloses nasal drops comprising 0,10% w/w xylometazoline HCl, 2,00% w/w sorbitol, 0,40% w/w sodium chloride, 0,50% w/w HPMC and 97,17% w/w water.

However, known nasal drops and sprays often rapidly exit the nasal cavity either via dripping from the nostrils or via the back of the nasal cavity into the nasopharynx, which can lead to insufficient efficacy of the physiologically active agent(s). High-viscosity delivery systems, such as ointments or gels, are retained in the nasal cavity for a longer time period, but the exact dosage of ointments and gels is difficult to meter and subsequently deliver to the desired location within the nasal cavity. Similar problems are experienced if pharmaceutical compositions are applied to other mucosae, such as the mucous membrane of the eyes or to mucosae in the oral cavity, such as the buccal mucosa.

To address this problem, European Patent EP 0 023 359 discloses a powdery pharmaceutical composition for application to the mucosa of the nasal cavity which comprises a drug and a carrier. At least 90% of the composition consists of particles having an effective particle diameter of 20 to 250 µm. The composition comprising a lower alkyl ether of cellulose having a viscosity, determined at 37°C±0.2°C for a 2% aqueous solution thereof, of 5 to 5000 mPa•s. The lower alkyl ether of cellulose is preferably methyl cellulose, hydroxypropyl cellulose or hydroxypropylmethyl cellulose. Those having a degree of ether substitution of 0.1 to 6, especially 0.4 to 4.6 are said to be preferred. The pharmaceutical composition absorbs mucus on the nasal mucosa and covers the nasal mucosa as a fluid surface. Unfortunately, spraying a powdery pharmaceutical composition into the nasal cavity is quite complex. The European Patent EP 023 359 suggests filling a capsule with the powdery composition, mounting it in a sprayer equipped by a needle, piercing the capsule with the needle to provide minute holes on the top and bottom sides of the capsule and thereafter sending air by means of a rubber ball to jet out the powder. Moreover, a powdery composition often gives the feel of foreign matter in the nasal cavity, irritates the nasal cavity and can lead to drying out of the nasal cavity.

In view of the above-mentioned deficiencies of the prior art compositions to be applied to a mucosa, the object of the present invention is to provide a composition that can be easily applied onto a mucosa, and that is retained on the mucosa for an extended time period.

### SUMMARY

One aspect of the present invention is a composition for application to a mucosa which comprises 0.1 to 10 percent of a tonicity-adjusting agent, from 0.5 to 10 percent of a hydroxyalkyl methylcellulose, from 0 to 20 percent of a physiologically active agent, and from 0 to 30 percent of one or more adjuvants, based on the total weight of the composition, the remainder being a liquid diluent, wherein the hydroxyalkyl methylcellulose has a DS of from 1.6 to 2.7 and an MS of from 0.40 to 1.30, wherein DS is the degree of substitution of methoxyl groups and MS is the molar substitution of hydroxyalkoxyl groups, and at least 55 weight percent of the liquid diluent is water.

Another aspect of the present invention is a container which comprises the above-mentioned composition, wherein the container is designed for releasing the composition by spraying or as drops.

Yet another aspect of the present invention is a method of transmucosal administration of a physiologically active agent to an individual wherein the above-mentioned composition, that additionally comprises a physiologically active agent, is applied to a mucosa of the individual.

### BRIEF DESCRIPTION OF DRAWINGS

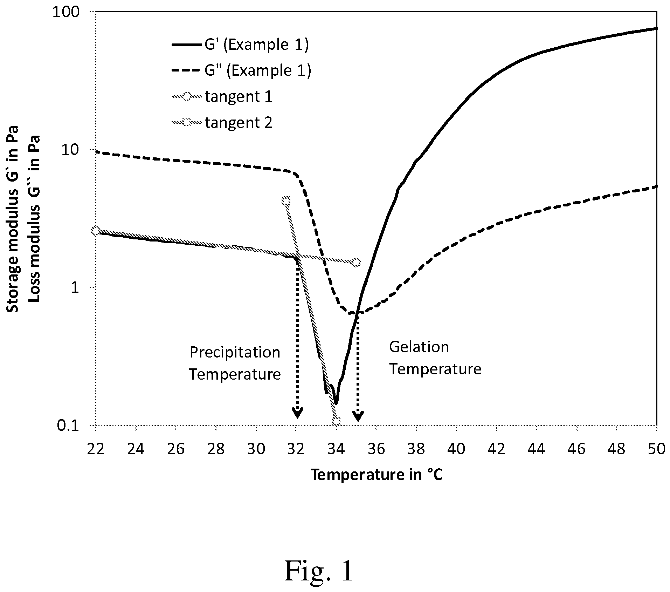

Fig. 1 illustrates how to determine the precipitation temperature and the gelation temperature of a composition of the present invention.

### DESCRIPTION OF EMBODIMENTS

Surprisingly, it has been found that the composition of the present invention exhibits a precipitation temperature of up to 37 °C, typically up to 35 °C, and more typically up to 33 °C. Generally, the composition of the present invention also exhibits a gelation temperature of up to 37 °C, typically up to 35 °C. The precipitation temperature of the composition of the present invention is generally at least 12 °C, typically at least 15 °C, more typically at least 20 °C, and most typically at least 24 °C. The gelation temperature of the composition of the present invention is generally at least 24 °C, typically at least 26 °C, more typically at least 28 °C, and most typically at least 30 °C.

The composition of the present invention is very useful for application to a mucosa, e.g. for transmucosal delivery of a physiologically active agent. A low viscosity at 5°C or 20 °C, i.e., at a temperature at which the composition is usually stored and/or applied, facilitates the release of the composition from a container comprising such composition, e.g. as drops or by spraying, and the administration of the composition to a mucosa. The temperature of the composition increases after its application to a mucosa.

At the precipitation temperature, the hydroxyalkyl methylcellulose starts to precipitate from solution, which increases the adherence of the hydroxyalkyl methylcellulose and, accordingly, of the tonicity-adjusting agent and the typically present physiologically active agent, to the mucosa. The hydroxyalkyl methylcellulose, the tonicity-adjusting agent and the typically present physiologically active agent are concentrated into the small precipitated layer most intimately interfacing with the mucosa. The precipitation event occurs at a lower temperature than the thermal gelation event.

Thermal gelation at the gelation temperature of the composition of the present invention triggers an additional phenomenon to maximize efficacy of the delivery system: The high-viscosity major portion of the thermally gelled composition facilitates retention of the composition of the present invention on the mucosa. Conventionally, hydroxyalkyl methylcellulose, particularly hydroxypropyl methylcellulose, has been found to be very useful in a variety of applications, providing thickening, freeze/thaw stability, lubricity, moisture retention and release, film formation, modified-release, texture, consistency, shape retention, emulsification, binding, gelation, and suspension properties. However, hydroxyalkyl methylcelluloses, such as hydroxypropyl methylcellulose, usually do not provide a sufficient thickening effect to compositions at temperatures encountered within the nasal cavities of mammals, such as those of human beings, as shown in the accompanying examples. One unusual property of hydroxyalkyl methylcelluloses is that they are known to exhibit reverse thermal gelation in water; in other words, hydroxyalkyl methylcellulose gels at temperatures above 50 °C when dissolved at a concentration of 2% and forms a liquid if cooled again down to temperatures of 20 °C or less. Most grades of hydroxyalkyl methylcellulose, dissolved alone to 2 wit.% in water at about 5 °C, will precipitate and subsequently gel at least about 10 °C higher than the normal body temperature of a human being.

In the present invention, a specific hydroxyalkyl methylcellulose is an essential component of the composition for transmucosal delivery. Hydroxypropyl methylcelluloses are derivatives of cellulose. Cellulose consists of β-1,4 glycosidically bound D-glucopyranose repeating units, designated as anhydroglucose units in the context of this invention, which are represented for unsubstituted cellulose by the formula 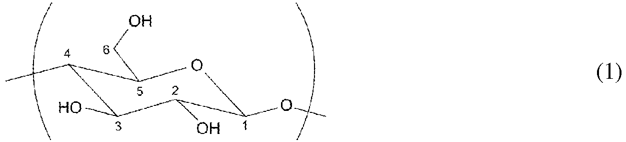 illustrating the numbering of the carbon atoms in the anhydroglucose units. The numbering of the carbon atoms in the anhydroglucose units is referred to in order to designate the position of ether substituents covalently bound to the respective carbon atom. In the hydroxyalkyl methylcellulose, at least a part of the hydroxyl groups of the cellulose backbone at the 2-, 3- and 6-positions of the anhydroglucose units are substituted by a combination of methoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the hydroxyalkyl methylcellulose. Preferably a single kind of hydroxyalkoxyl group, more preferably hydroxypropoxyl, is present. Illustrative of the hydroxyalkyl methylcelluloses are hydroxyethyl methylcelluloses, hydroxypropyl methylcelluloses, and hydroxybutyl methylcelluloses. Most preferably, the hydroxyalkyl methylcellulose is a hydroxypropyl methylcellulose. The hydroxyl groups of the cellulose backbone at the 2-, 3- and 6-positions of the anhydroglucose units are not substituted by any groups other than methoxyl and hydroxyalkoxyl groups.

An essential feature of the specific hydroxyalkyl methylcellulose used in the composition of the present invention is the degree of the substitution of hydroxyl groups at the 2-, 3- and 6-positions of the anhydroglucose units by methoxyl groups and hydroxyalkoxyl groups. The average number of methoxyl groups per anhydroglucose unit is designated as the degree of substitution of methoxyl groups, DS. In the definition of DS, the term "hydroxyl groups substituted by methoxyl groups" is to be construed within the present invention to include not only methylated hydroxyl groups directly bound to the carbon atoms of the cellulose backbone, but also methylated hydroxyl groups of hydroxyalkoxyl substituents bound to the cellulose backbone.

The degree of the substitution of hydroxyl groups at the 2-, 3- and 6-positions of the anhydroglucose units by hydroxyalkoxyl groups is expressed by the molar substitution of hydroxyalkoxyl groups, the MS. The MS is the average number of moles of hydroxyalkoxyl groups per anhydroglucose unit in the hydroxyalkyl methylcellulose. It is to be understood that during the hydroxyalkylation reaction the hydroxyl group of a hydroxyalkoxyl group bound to the cellulose backbone can be further etherified by a methylation agent and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone. The term "hydroxyalkoxyl groups" thus has to be interpreted in the context of the MS as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further methylated or not; both methylated and non-methylated hydroxyalkoxyl substituents are included for the determination of MS.

The hydroxyalkyl methylcellulose utilized in the composition of the present invention has a DS of from 1.6 to 2.7 and an MS of from 0.40 to 1.30. Preferably the hydroxyalkyl methylcellulose has a DS of from 1.6 to 2.3, more preferably from 1.70 to 2.20. Preferably the hydroxyalkyl methylcellulose has an MS of from 0.50 to 1.20, more preferably from 0.60 to 1.10. Any preferred range for DS can be combined with any preferred range for MS. Most preferably the hydroxyalkyl methylcellulose has a DS of from 1.70 to 2.20 and an MS of from 0.60 to 1.10. The sum of the DS and MS preferably is at least 2.0, more preferably at least 2.3, most preferably at least 2.4 and preferably up to 3.2, more preferably up to 3.0, most preferably up to 2.9.

The degree of substitution of methoxyl groups (DS) and the molar substitution of hydroxyalkoxyl groups (MS) can be determined by Zeisel cleavage of the hydroxyalkyl methylcellulose with hydrogen iodide and subsequent quantitative gas chromatographic analysis (G. Bartelmus and R. Ketterer, Z. Anal. Chem., 286 (1977) 161-190). When the hydroxyalkyl methylcellulose is hydroxypropyl methylcellulose, the determination of the % methoxyl and % hydroxypropoxyl is carried out according to the United States Pharmacopeia (USP 35, "Hypromellose", pages 3467-3469). The values obtained are % methoxyl and % hydroxypropoxyl. These are subsequently converted into degree of substitution (DS) for methoxyl substituents and molar substitution (MS) for hydroxypropoxyl substituents. Residual amounts of salt have been taken into account in the conversion.

The hydroxyalkyl methylcellulose utilized in the composition of the present invention preferably has a viscosity of from 3 to 200,000 mPa·s, more preferably from 20 to 200,000 mPa·s, even more preferably from 20 to 50,000 mPa·s, most preferably from 20 to 15,000 mPa·s, and particularly from 40 to 5,000 mPa·s, measured as a 2 weight-% solution in water at 20 °C. Viscosities of up to 600 mPa·s are determined by Ubbelohde viscosity measurement. Viscosities above 600 mPa·s are determined using a Brookfield viscometer. Descriptions on preparing the 2 wt. % HPMC solution and both Ubbelohde and Brookfield viscosity measurement conditions are described in the United States Pharmacopeia (USP 35, "Hypromellose", pages 423 - 424 and 3467-3469 and in ASTM D-445 and ISO 3105 referenced therein).

Another essential ingredient of the composition of the present invention is a tonicity-adjusting agent. One or more tonicity-adjusting agents may be included in the composition of the present invention to partially or fully achieve tonicity with body fluids, e.g. fluids of the nasal cavity or fluids of the eye, resulting in reduced levels of irritation. Examples of pharmaceutically acceptable tonicity-adjusting agents include, but are not limited to, sodium chloride, potassium chloride, dextrose, xylitol, calcium chloride, glucose, glycerin, mannitol, and sorbitol. A tonicity-adjusting agent is included in an amount of from 0.1 to 10 percent, preferably from 0.2 to 8.0 percent, more preferably from 0.3 to 6.0 percent, and most preferably from 0.5 to 4.0 percent, based on the total weight of the composition. In one embodiment, the tonicity-adjusting agent is dextrose and/or xylitol. In another embodiment, the tonicity-adjusting agent is sodium chloride. In yet another embodiment the tonicity-adjusting agent is a buffering agent. Suitable buffering agents include, but are not limited to, organic acid salts such as salts of citric acid, gluconic acid, carbonic acid, tartaric acid, succinic acid, acetic acid, phthalic acid, or phosphoric acid. A phosphate buffer is particularly useful. Phosphate buffers typically comprise sodium or potassium phosphate dibasic or sodium or potassium phosphate monobasic. In addition, amino acid components can also be used as buffering agent. Such amino acid component includes without limitation glycine and histidine. The buffering agent provides improved pH control. In one embodiment, the composition of the invention has a pH between 5.0 and 8.0, preferably between 6.0 and 8.0, and more preferably between 6.0 and 7.0. In a specific embodiment, a composition of the invention has a pH of about 6.5.

It has surprisingly been found that a composition of the present invention which comprises the hydroxyalkyl methylcellulose described further above in combination with a tonicity-adjusting agent exhibits thermal precipitation and/or thermal gelation at a lower temperature than a comparable composition containing the same type and amount of hydroxyalkyl methylcellulose without the tonicity-adjusting agent. Alternatively, a lower concentration of the afore-mentioned hydroxyalkyl methylcellulose and/or an afore-mentioned hydroxyalkyl methylcellulose having a lower viscosity (measured as a 2 wt-% solution in water at 20 °C) can be utilized in the presence of a tonicity-adjusting agent while still achieving thermal precipitation and/or thermal gelation of the composition at the desired temperature. The effects of the tonicity-adjusting agent in combination with the hydroxyalkyl methylcellulose described further above are illustrated in more detail in the Examples.

The composition of the present invention is useful for application to mucosae, e.g., for intranasal, buccal, sublingual, vaginal, ocular or rectal application.

In one embodiment of the invention the composition comprises one or more physiologically active agents, preferably one or more drugs, one or more diagnostic agents, or one or more essential oils, or one or more physiologically active agents which are useful for cosmetic or nutritional purposes. The term "drug" denotes a compound having beneficial prophylactic and/or therapeutic properties when administered to an individual, typically a mammal, especially a human individual. Physiologically active agents that are useful for transmucosal delivery, such as intranasal, buccal, sublingual, vaginal, ocular or rectal delivery, or delivery through a mucosal membrane located on the gums or lips are known in the art.

The composition of the present invention is particularly useful for intranasal delivery of one or more physiologically active agents or for delivery through a mucosal membrane located in the oral cavity, such as drugs utilized in therapies for allergic rhinitis, nasal congestion and infections, in treatments of diabetes, migraine, nausea, smoking cessation, acute pain relief, nocturnal enuresis, osteoporosis, vitamin B-12 deficiency and for administering intranasal influenza vaccine, however the physiologically active agents are not limited to these examples. Especially preferred drugs are acetaminophen, azelastine hydrochloride, beclomethasone dipropionate monohydrate, sumatriptan succinate, dihydroergotamine mesylate, fluticasone propionate, triamcinolone acetonide, budesonide, fentanyl citrate, butorphanol tartrate, zolmitriptan, desmopressin acetate hydrate, salmon calcitonin, nafarelin acetate, buserelin acetate, elcatonin, oxytocin, insulin, mometasone furoate, estradiol, metoclopramide, xylometazoline hydrochloride, ipratropium bromide hydrate, olopatadine hydrochloride, oxymetazoline hydrochloride, dexpanthenol, hydrocortisone, naphazoline hydrochloride, phenylephrine hydrochloride, mepyramine maleate, phenylephrine hydrochloride, cromolyn sodium, levocabastine hydrochloride, vitamin B12, prednisolone sodium metasulphobenzoate, naphazoline nitrate, tetrahydrozoline hydrochloride, chlorpheniramine maleate, benzethonium chloride, ketotifen fumarate, histamine dihydrochloride, fusafungine, or combinations thereof. Examples of essential oils are menthol, methyl salicylate, thymol, eucalyptus oil, camphor, anise, sweet orange, or combinations thereof. It has surprisingly been found that the presence of certain physiologically active agents in the composition of the present invention can further reduce the temperature at which thermal precipitation and/or thermal gelation occurs.

In yet another embodiment of the invention the composition does not comprise a physiologically active agent that is selected from drugs, diagnostic agents, essential oils, or physiologically active agents which are useful for cosmetic or nutritional purposes. Compositions comprising an above-described hydroxyalkyl methylcellulose in combination with a tonicity-adjusting agent but not a physiologically active agent in addition are useful, e.g., for rinsing and/or moisturizing the nasal cavity or as artificial tears.

The composition for transmucosal delivery further comprises a liquid diluent, of which at least 55 weight percent and up to 100 percent is water. The composition of the present invention may additionally comprise an organic liquid diluent; however, the composition of the present invention should comprise at least 55, preferably at least 65, more preferably at least 75, most preferably at least 90, and particularly at least 95 weight percent of water and up to 45, preferably up to 35, more preferably up to 25, most preferably only up to 10, and particularly only up to 5 weight percent of an organic liquid diluent, based on the total weight of the organic liquid diluent and water. In one embodiment the diluent consists of water. The water is typically a high-quality grade of water such as purified water, for example USP purified water, PhEur purified water or water for Injection (WFI).

The term "organic liquid diluent" as used herein means an organic solvent or a mixture of two or more organic solvents that is liquid at 25 °C and atmospheric pressure. Preferred organic liquid diluents are polar organic solvents having one or more heteroatoms, such as oxygen, nitrogen or halogen (like chlorine). More preferred organic liquid diluents are alcohols, for example multifunctional alcohols, such as propylene glycol, polyethylene glycol, polypropylene glycol and glycerol; or preferably monofunctional alcohols, such as ethanol, isopropanol or n-propanol; or acetates, such as ethyl acetate. More preferably the organic liquid diluents have 1 to 6, most preferably 1 to 4 carbon atoms. The organic liquid diluent is preferably pharmaceutically acceptable, such as ethanol or glycerol.

The composition of the present invention may comprise one or more optional adjuvants, such as one or more suspending agents, odor, flavor or taste improvers, preservatives, pharmaceutically acceptable surfactants, coloring agents, opacifiers, or antioxidants. Typically, pharmaceutically acceptable optional adjuvants are selected.

For stability purposes, compositions of the invention (for example intranasal compositions) may be protected from microbial or fungal contamination and growth by inclusion of one or more preservatives. Examples of pharmaceutically acceptable anti-microbial agents or preservatives may include, but are not limited to, quaternary ammonium compounds (e.g. benzalkonium chloride, benzethonium chloride, cetrimide, cetylpyridinium chloride, lauralkonium chloride and myristyl picolinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium edetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives may include, but are not limited to, sodium benzoate, sorbic acid, sodium propionate, methylparaben, ethylparaben, propylparaben and butylparaben. The preservative(s), if included, are typically present in an amount of from 0.001 to 1%, such as from 0.015% to 0.5%, based on the total weight of the composition. In one embodiment, the preservative is selected from benzalkonium chloride, EDTA and/or potassium sorbate. In a further embodiment, the preservative is EDTA and/or potassium sorbate.

Typically the composition of the present invention comprises from 0.5 to 10 percent, and most preferably from 0.5 to 8 percent of the hydroxyalkyl methyl cellulose defined above, from 0.1 to 10 percent, more preferably from 0.2 to 8.0 percent, even more preferably from 0.3 to 6.0 percent, and most preferably from 0.5 to 4.0 percent of a tonicity-adjusting agent, from 0 to 20 percent, or from 0.01 to 10 percent, or from 0.1 to 5 percent of a physiologically active agent, and from 0 to 30 percent, or from 0.01 to 20 percent, or from 0.1 to 10 percent, of one or more optional adjuvants, based on the total weight of the composition, the remainder being a liquid diluent. At least 55 percent, preferably at least 65 percent, more preferably at least 75 percent, most preferably at least 90 percent, and particularly at least 95 percent and up to 100 percent of the weight of the liquid diluent is water. The composition generally comprises a sufficient amount of liquid diluent that the composition is liquid at 5 °C. Preferably, the amount of the liquid diluent is at least 50 percent, more preferably at least 80 percent, and most preferably at least 90 percent, based on the total weight of the composition. The composition of the present invention preferably has a viscosity of from 100 to 50,000 mPa·s, more preferably from 100 to 30,000 mPa·s, even more preferably from 200 to 10,000 mPa·s, and most preferably from 500 to 4000 mPa·s, measured at 5 °C at a shear rate of 10 s⁻¹.

The composition of the present invention is preferably in the form of a sprayable solution or suspension, a solution or suspension to be applied as drops or in the form of a syrup. The composition can be packaged into suitable containers, which can also serve as delivery devices, e.g., containers designed for generating and subsequently applying sprays or drops to the intended delivery site. The delivery devices can be multi-dose or unit-dose devices.

The composition of the present invention is preferably packaged in a container such that the volume of the composition in the container is not more than 100 ml, more preferably not more than 50 ml or not more than 25 ml. Typically the volume of the composition in the container is at least 0.1 ml, or at least 1 ml, or at least 2 ml, or at least 5 ml, or at least 10 ml. The volume of the liquid composition typically depends on the intended use. Single-dose vials often comprise only 0.1 - 2 ml of a liquid composition. Sprays or bottles which are designed to release the composition drop by drop usually comprise larger amounts of the liquid composition, e.g., 5 - 50 ml or 10 - 25 ml.

The composition of the present invention is preferably stored at a temperature of from 1 to 23 °C, more preferably from 5 to 20 °C. Upon application of the composition of the present invention to a mucosa of an individual, the temperature of the composition increases and the hydroxyalkyl methylcellulose suspended or, preferably, dissolved in the aqueous diluent of the composition precipitates or gels when the temperature of the composition of the present invention adjusts to the temperature of the mucosa, i.e., to a temperature of 30 - 37 °C, typically 30 - 35°C. The exact temperature of the mucosa somewhat depends on the type of mucosa, on the individual, on the time of day, and on the conditions of the surrounding environment. In the case of human beings the mucosa in the nasal cavity typically has a temperature of 30 - 35°C, the oral mucosa under the tongue typically has a temperature of about 36.8 ± 0.4°C, and the rectal mucosa typically has a temperature of about 37°C. At the precipitation temperature, the hydroxyalkyl methylcellulose starts to precipitate from solution, which increases the adherence of the hydroxyalkyl methylcellulose and, accordingly, of the tonicity-adjusting agent and the typically present physiologically active agent, to the mucosa. The hydroxyalkyl methylcellulose, the tonicity-adjusting agent and the typically present physiologically active agent are concentrated into the small precipitated layer most intimately interfacing with the mucosa. The precipitation of the hydroxyalkyl methylcellulose occurs immediately prior to the thermal gelation event (see Figure 1).

The subsequent thermal gelation of the composition leads to an additional event, which maximizes efficacy of the delivery system: an increased viscosity of the majority of the composition of the present invention, which causes the composition to set and be retained at the deposition site, e.g., in the nasal or oral cavity.

The precipitation or gelation of the composition can be determined as described in the Examples section. The composition of the present invention exhibits a precipitation temperature of up to 37 °C, typically up to 35 °C, and more typically up to 33 °C. Due to its low precipitation temperature, the composition of the present invention is particularly useful for application to the nasal mucosa. Generally, the composition of the present invention also exhibits a gelation temperature of up to 37 °C, typically up to 35 °C.

The precipitation and gelation behavior of the hydroxyalkyl methylcellulose utilized in the compositions of the present invention can be adapted to a certain degree to the specific need of a certain transmucosal delivery system. E.g., the desired viscosity increase of the composition of the present invention can be achieved at a lower temperature if a hydroxyalkyl methylcellulose of higher viscosity grade, measured as a 2 weight-% solution in water at 20 °C, is selected than when a hydroxyalkyl methylcellulose of lower viscosity grade is selected. Also, the desired viscosity increase of the composition of the present invention can be achieved at a lower temperature when the composition has a higher concentration of the hydroxyalkyl methylcellulose than when its concentration is lower.

It has surprisingly been found that thermal precipitation and thermal gelation of the composition of the present invention can be achieved at even a lower temperature in the presence of a physiologically active agent as described above. The effects of the physiologically active agent in combination with the hydroxyalkyl methylcellulose described further above and a tonicity-adjusting agent are illustrated in more detail in the Examples. Some embodiments of the invention will now be described in detail in the following Examples.

### EXAMPLES

Unless otherwise mentioned, all parts and percentages are by weight. In the Examples the following test procedures are used.

### Determination of the DS and MS of hydroxypropyl methylcellulose

The determination of the % methoxyl and % hydroxypropoxyl was carried out according to the United States Pharmacopeia (USP 35, "Hypromellose", pages 3467-3469). The values obtained were % methoxyl and % hydroxypropoxyl. These were subsequently converted into degree of substitution (DS) for methoxyl substituents and molar substitution (MS) for hydroxypropoxyl substituents. Residual amounts of salt were taken into account in the conversion.

### Determination of the viscosity of hydroxypropyl methylcellulose

The viscosity of the hydroxypropyl methylcellulose (HPMC) samples was measured as a 2.0 % by weight solution in water at 20°C ± 0.1 °C. Viscosities of up to 600 mPa·s were determined by Ubbelohde viscosity measurement. Viscosities above 600 mPa·s were determined using a Brookfield viscometer. Descriptions on preparing the 2 wt. % HPMC solution and both Ubbelohde and Brookfield viscosity measurement conditions are described in the United States Pharmacopeia (USP 35, "Hypromellose", pages 423 - 424 and 3467 - 3469 and in ASTM D-445 and ISO 3105 referenced therein).

### Determination of the viscosity of aqueous solutions for application to a mucosa

The viscosities of aqueous solutions for application to a mucosa were measured using an ARES RFS3 rheometer with cup and bob fixtures (TA-Instruments) at 5 °C and at a shear rate of 10 s⁻¹.

### Determination of Storage Modulus G', Loss Modulus G", Precipitation Temperature and Gelation Temperature

Rheology measurements of aqueous solutions comprising HPMC and optionally a tonicity-adjusting agent, such as a buffering agent, and/or a physiologically active agent were conducted with an Ares RFS3 rheometer (TA-Instruments) with cup and bob fixtures. Seventeen milliliters of solution were transferred to the cup fixture and the gap set to 5 mm. The sample was heated at a rate of 1°C per minute with a constant strain of 2% and a constant angular frequency of 5 radians per second. The storage modulus G', which was obtained from the rheology measurements, represents the elastic properties of the solution. The loss modulus G", which was obtained from the rheology measurements, represents the viscous properties of the solution.

When precipitation of the HPMC took place at elevated temperatures the storage modulus dropped. This precipitation temperature was analyzed from a plot of the log storage modulus G' vs. temperature as the cross-over of two tangents. The first tangent was fitted to the decrease of the storage modulus G' with increasing temperatures and the second tangent was fitted to the drop of the storage modulus over a temperature region of 1 - 3 °C. The precipitation temperature is an important parameter for aqueous solutions which are designed for application to a mucosa. With further increasing temperatures the storage modulus values were increasing and a cross-over between the storage modulus and the loss modulus was obtained. The cross-over of G' and G" is determined to be the gelation temperature.

Fig. 1 illustrates how to determine the precipitation temperature and the gelation temperature of the composition of the present invention and of comparative compositions. The results for Example 1 are illustrated in Fig. 1.

### Utilized HPMCs

METHOCEL™ E4M, METHOCEL™ F4M and METHOCEL™ K4M cellulose ethers used in Comparative Examples A-C are commercially available from The Dow Chemical Company.

Hydroxyalkyl methylcelluloses having a DS of from 1.6 to 2.7 and an MS of from 0.40 to 1.30 are known from U.S. Patent No. 4,614,545. Such HPMCs are used in the present invention and are represented by HPMC-I, HPMC-II and HPMC-III. Hydroxyalkyl methylcelluloses having the indicated DS and MS and a reduced molecular weight, such as HPMC-II and HPMC-III, can be produced by partial depolymerization of a higher molecular weight HPMC as described in European patent application EP 1 141 029 and the prior art cited therein.

**Table 1**

| HPMC | Abbreviation | DS | MS | HPMC viscosity as 2.0 wt. % aqueous solution 20°C |
|---|---|---|---|---|
| HPMC-I | | 1.97 | 0.84 | 2698 |
| HPMC-II | | 2.03 | 0.81 | 97 |
| HPMC-III | | 2.03 | 0.87 | 44 |
| METHOCEL™ E4M | E4M | 1.93 | 0.23 | 5045 |
| METHOCEL™ F4M | F4M | 1.88 | 0.15 | 4970 |
| METHOCEL™ K4M | K4M | 1.45 | 0.22 | 5494 |

### Preparation of aqueous solutions for application to the nasal mucosa

Concentrated HPMC solutions were prepared by dispersing the HPMC into water of a temperature of greater than 60 °C while stirring using a Yamato LT 400 lab overhead mixer to achieve a good dispersion at 400 rpm. Concentrated solutions containing 2.5 wt. % HPMC-I, METHOCEL™ E4M, METHOCEL™ F4M or METHOCEL™ K4M were prepared. Concentrated solutions containing 7.0 wt. % HPMC-II, and concentrated solutions containing 10.0 wt. % HPMC-III were prepared. The solutions were cooled and stirred until room temperature until the HPMC was fully dispersed. Then the container comprising the dispersion was placed in an ice bath having a temperature of less than 5 °C and stirring continued for 1 hour. The HPMC solution was then equilibrated overnight in a refrigerator at 5 °C prior to use.

A concentrated HPMC solution from the refrigerator was mixed either with water or with a concentrated aqueous solution of a tonicity-adjusting agent and/or an active pharmaceutical ingredient (API) from the refrigerator by the use of a stirrer until a homogenous solution was achieved. Acetaminophen was used as API. During this additional mixing the solution temperature was still cold and a temperature above 7 °C was not reached. In cases where the concentration of the API in water was greater than its solubility, a suspension of the API was prepared and the HPMC solution was added to this suspension while mixing. The mixing ratio of the two solutions was chosen to provide the desired HPMC concentration in the resulting mixture. The concentration of the tonicity-adjusting agent and/or the API in the concentrated aqueous solution was chosen to achieve the desired concentration in the resulting mixture at the chosen mixing ratio. For example, the aqueous solution of Example 1 was prepared by mixing 4 volume parts of an aqueous solution of 2.5 wt. % HPMC-I with 1 volume part of an aqueous solution of 15 wt. % NaCl. The final compositions of all Examples and Comparative Examples were fully soluble, clear solutions.

### Example 1 and Comparative Examples A - C

The procedure described above was applied to prepare aqueous solutions comprising 2 weight percent of an HPMC as listed in Table 2 below and 3 weight percent of NaCl, based on the total weight of the aqueous solution. The remainder was water. The precipitation temperatures and the gelation temperatures are listed in Table 2 below.

**Table 2**

| (Comparative) Example | Aqueous Solution | Precipitation temperature, °C | Gelation temperature, °C |
|---|---|---|---|
| 1 | 2% HPMC-I, 3% NaCl | 32.0 | 35.0 |
| A | 2% E4M, 3% NaCl | 46.3 | 44.0 |
| B | 2% F4M, 3% NaCl | 46.6 | 51.9 |
| C | 2% K4M, 3% NaCl | Not detected | 56.5 |

The results in Table 2 illustrate that the HPMC-I of Example 1 has substantial advantages over comparative HPMCs. When incorporating HPMC-I in combination with a tonicity-adjusting agent in an aqueous composition, precipitation takes place at a temperature as low as 32 °C, which is about the temperature of the mucosa in the human nose. Precipitation at a temperature as low as 32 °C is very advantageous for intranasal application of the composition. Moreover, the aqueous solution of the present invention according to Example 1 already forms a gel which falls within the normal temperature range of the nasal cavity and is also below the normal temperature of the oral cavity. The aqueous solutions of Comparative Examples A - C exhibit precipitation and gelation temperatures which are far above the temperature of the nasal or oral mucosa of human beings and do not have an improved adherence to such mucosae. Therefore, the HPMCs of Comparative Examples A to C are much less suitable than the HPMC-I of Example 1 in compositions which are to be applied to a mucosa.

### Examples 2a and 2b, 3a and 3b

Table 3 below illustrates aqueous solutions of the present invention comprising HPMC-II at various concentrations, 3% NaCl and the remainder being water. The solutions were produced and measured twice. The repetitions show good reproducibility.

**Table 3**

| Example | Aqueous Solution | Precipitation temperature, °C | Gelation temperature, °C |
|---|---|---|---|
| 2a | 6% HPMC-II, 3% NaCl | 32.0 | 35.6 |
| 2b | 6% HPMC-II, 3% NaCl | 32.7 | 36.2 |
| 3a | 4% HPMC-II, 3% NaCl | 32.7 | 36.1 |
| 3b | 4% HPMC-II, 3% NaCl | 32.9 | 36.8 |

### Examples 2a, 2b, 4 - 8 and Comparative Examples D - F

Tables 4 - 6 below illustrate aqueous solutions comprising various HPMCs within the scope of the present invention of various viscosity grades and at various concentrations in the absence or presence of a tonicity-adjusting agent (NaCl) and optionally a physiologically active agent, the remainder being water.

**Table 4**

| (Comparative) Example | Aqueous Solution | Precipitation temperature, °C | Gelation temperature, °C |
|---|---|---|---|
| D | 6% HPMC-II | 40.8 | 45.3 |
| 2a | 6% HPMC-II, 3% NaCl | 32.0 | 35.6 |
| 2b | 6% HPMC-II, 3% NaCl | 32.7 | 36.2 |
| 4 | 6% HPMC-II, 3% NaCl, 1% acetaminophen | 24.4 | 28.4 |

**Table 5**

| (Comparative) Example | Aqueous Solution | Precipitation temperature, °C | Gelation temperature, °C |
|---|---|---|---|
| E | 2% HPMC-I | 40.5 | 43.8 |
| 5 | 2% HPMC-I, 3% NaCl | 32.0 | 35.0 |
| 6 | 2% HPMC-I, 3% NaCl, 1% acetaminophen | 17.8 | 26.7 |

**Table 6**

| (Comparative) Example | Aqueous Solution | Precipitation temperature, °C | Gelation temperature, °C |
|---|---|---|---|
| F | 8% HPMC-III | 40.1 | 44.5 |
| 7 | 8% HPMC-III, 3*%* NaCl | 30.8 | 33.7 |
| 8 | 8% HPMC-III, 3*%* NaCl, 1% acetaminophen | 15.0 | 27.2 |

The results in Tables 4 - 6 illustrate that surprisingly an aqueous solution which comprises an HPMC having a DS and MS within the scope of the present invention in combination with a tonicity-adjusting agent has a considerably lower precipitation temperature and gelation temperature than a corresponding aqueous solution which comprises the same HPMC in the absence of a tonicity-adjusting agent. The precipitation temperature of the compositions of Examples 2a and 2b, 5 and 7 is low enough that precipitation on the nasal mucosa takes place. Moreover, a physiologically active agent, e.g., a drug like acetaminophen, lowers the precipitation temperature and gelation temperature of the aqueous solution even more, as illustrated by Examples 4, 6 and 8. Accordingly, the compositions of the present invention are very useful for application to a mucosa, particularly to the nasal mucosa, where the HPMC precipitates and facilitates the transmucosal delivery of other ingredients in the composition, such as drugs.

### Examples 9 - 11

Table 7 below illustrates HPMCs having a DS and MS within the scope of the present invention of various viscosities at various concentrations in a pH 6.5 phosphate buffered solution (PBS) which comprises 0.65 weight percent sodium chloride and 0.25 weight percent sodium phosphate in deionized water. Each of the solutions additionally comprises 1% acetaminophen.

**Table 7**

| Example | Aqueous Solution | Precipitation temperature, °C | Viscosity of aq. solution at 5°C, 10 s⁻¹, mPa·s |
|---|---|---|---|
| 9 | 8% HPMC-III, PBS, 1% acetaminophen | 32.2 | 17830 |
| 10 | 6% HPMC-II, PBS, 1% acetaminophen | 30.5 | 10230 |
| 11 | 2% HPMC-I, PBS, 1% acetaminophen | 30.6 | 3350 |

The results in Table 7 illustrate that compositions for transmucosal delivery of a physiologically active agent, such as a drug like acetaminophen, can be prepared wherein the HPMC in the compositions precipitates at or below the normal temperature of the nasal or oral mucosa of human beings. Precipitation increases the adherence of the hydroxyalkyl methylcellulose and, accordingly, of the tonicity-adjusting agent and the drug to the mucosa. The hydroxyalkyl methylcellulose, the tonicity-adjusting agent and the drug are concentrated into the small precipitated layer most intimately interfacing with the mucosa. Below the precipitation temperature, e.g. at a typical refrigerator temperature (5°C) or at typical room temperature (20 °C) the solutions have a lower viscosity, which facilitates the release of the composition from a container comprising such composition, e.g. as drops or by spraying, and the administration of the composition to a mucosa. The results in Table 7 illustrate that compositions of the present invention can be prepared which have a range of viscosities at refrigerator or room temperature. This allows adaptation of the compositions of the present invention to the desired end-use. E.g., the compositions of Examples 9 and 10 are mainly suitable as nasal drops whereas the composition of Example 11 can be used as nasal spray or as nasal drops. The relatively low viscosity of the composition of Example 11 facilitates spraying of the composition.

## Claims

1. A composition for application to a mucosa comprising from 0.1 to 10 percent of a tonicity-adjusting agent, from 0.5 to 10 percent of a hydroxyalkyl methylcellulose, from 0 to 20 percent of a physiologically active agent, and from 0 to 30 percent of one or more adjuvants, based on the total weight of the composition, the remainder being a liquid diluent, wherein
the hydroxyalkyl methylcellulose has a DS of from 1.6 to 2.7 and an MS of from 0.40 to 1.30, wherein DS is the degree of substitution of methoxyl groups and MS is the molar substitution of hydroxyalkoxyl groups, and
at least 55 weight percent of the liquid diluent is water.

2. The composition of claim 1 wherein the physiologically active agent is selected from one or more drugs, one or more diagnostic agents, one or more essential oils, or one or more physiologically active agents which are useful for cosmetic or nutritional purposes.

3. The composition of claim 1 or 2 wherein the hydroxyalkyl methylcellulose has a DS of from 1.70 to 2.20.

4. The composition of any one of claims 1 to 3 wherein the hydroxyalkyl methylcellulose has an MS of from 0.60 to 1.10.

5. The composition of any one of claims 1 to 4 wherein the hydroxyalkyl methylcellulose has a viscosity of from 20 to 50,000 mPa·s, measured as a 2 weight-% solution in water at 20 °C.

6. The composition of claim 5 wherein the hydroxyalkyl methylcellulose has a viscosity of from 40 to 5,000 mPa·s, measured as a 2 weight-% solution in water at 20 °C.

7. The composition of any one of claims 1 to 6 having a viscosity of from 100 to 50,000 mPa·s, measured at 5 °C.

8. The composition of claim 7 having a viscosity of from 200 to 10,000 mPa·s, measured at 5 °C and at a shear rate of 10 s⁻¹

9. The composition of any one of claims 1 to 8 exhibiting a precipitation temperature of from 12 to 37 °C.

10. The composition of any one of claims 1 to 9 for intranasal administration.

11. The composition of any one of claims 1 to 10 comprising from 0.5 to 10 percent of the hydroxyalkyl methyl cellulose, from 0.3 to 6.0 percent of a tonicity-adjusting agent, from 0.01 to 10 percent of a physiologically active agent, and from 0.01 to 20 percent of one or more adjuvants, based on the total weight of the composition, the remainder being the liquid diluent.

12. The composition of claim 2 wherein the essential oil is menthol, methyl salicylate, thymol, eucalyptus oil, camphor, anise, sweet orange, or a combination thereof.

13. The composition of any one of claims 1 to 12 comprising one or more tonicity-adjusting agents selected from sodium chloride, potassium chloride, dextrose, xylitol, calcium chloride, glucose, glycerin, mannitol and sorbitol.

14. A container comprising the composition of any one of claims 1 to 13,
wherein the container is designed to release the composition by spraying or as drops.

15. The composition of claims 1 to 13 for use in therapy or for use in therapies for allergic rhinitis, nasal congestion and infections or in treating diabetes, migraine, nausea, smoking cessation, acute pain relief, nocturnal enuresis, osteoporosis, vitamin B-12 deficiency or for administering intranasal influenza vaccine.

## Patentansprüche

1. Zusammensetzung für die Aufbringung auf eine Schleimhaut, enthaltend 0,1 bis 10 % eines Mittels zur Einstellung der Tonizität, 0,5 bis 10 % einer Hydroxyalkylmethylcellulose, 0 bis 20 % eines physiologisch aktiven Mittels, und 0 bis 30 % von einem oder mehreren Zusatzmitteln, bezogen auf das Gesamtgewicht der Zusammensetzung, der Rest ist flüssiges Verdünnungsmittel, wobei
die Hydroxyalkylmehtlycellulose einen DS von 1,6 bis 2,7 und einen MS von 0,40 bis 1,30 aufweist, wobei DS den Substitutionsgrad der Methoxygruppen und MS den molare Substitutionsgrad der Hydroxyalkoxygruppen darstellt, und
mindestens 55 Gew.-% des flüssigen Verdünnungsmittels Wasser ist.

2. Zusammensetzung nach Anspruch 1, wobei das physiologisch aktive Mittel ausgewählt ist aus einem oder mehreren Arzneimitteln, einem oder mehreren Diagnosemitteln, einem oder mehreren essentiellen Ölen oder einem oder mehreren physiologisch aktiven Mitteln, welche für Kosmetische- oder Ernährungszwecke verwendbar sind.

3. Zusammensetzung nach einem der Ansprüche 1 oder 2, wobei die Hydroxyalkylmethylcellulose einen DS von 1,70 bis 2,20 aufweist.

4. Zusammensetzung nach einem der Ansprüche 1 bis 3, wobei die Hydroxyalkylmethylcellulose einen MS von 0,60 bis 1,10 aufweist.

5. Zusammensetzung nach einem der Ansprüche 1 bis 4, wobei die Hydroxyalkylmethylcellulose eine Viskosität von 20 bis 50.000 mPa•s, gemessen als 2 gew.-%ige Lösung in Wasser bei 20 °C, aufweist.

6. Zusammensetzung nach Anspruch 5, wobei die
Hydroxyalkylmethylcellulose eine Viskosität von 40 bis 5.000 mPa•s, gemessen als eine 2 gew.-%ige Lösung in Wasser bei 20 °C, aufweist.

7. Zusammensetzung nach einem der Ansprüche 1 bis 6, welche eine Viskosität von 100 bis 50.000 mPa•s, gemessen bei 5 °C, aufweist.

8. Zusammensetzung nach Anspruch 7, welche eine Viskosität von 200 bis 10.000 mPa•s, gemessen bei 5 °C und einer Scherrate von 10 s⁻¹, aufweist.

9. Zusammensetzung nach einem der Ansprüche 1 bis 8, welche eine Fällungstemperatur von 12 bis 37 °C aufweist.

10. Zusammensetzung nach einem der Ansprüche 1 bis 9 für die intranasale Verabreichung.

11. Zusammensetzung nach einem der Ansprüche 1 bis 10, welche 0,5 bis 10 % der Hydroxyalkylmethylcellulose, 0,3 bis 6,0 % eines Mittels zur Einstellung der Tonizität, 0,01 bis 10 % eines physiologisch aktiven Mittels, und 0,01 bis 20 % von einem oder mehreren Zusatzmitteln, bezogen auf das Gesamtgewicht der Zusammensetzung, enthält, der Rest ist das flüssige Verdünnungsmittel.

12. Die Zusammensetzung nach Anspruch 2, wobei das essentielle Öl Menthol, Methylsalicylat, Thymol, Eukalyptusöl, Campher, Anis, Süßorange oder eine Kombination hiervon ist.

13. Zusammensetzung nach einem der Ansprüche 1 bis 12, enthaltend ein oder mehrere Mittel zur Einstellung der Tonizität, ausgewählt aus Natriumchlorid, Kaliumchlorid, Dextrose, Xylitol, Calciumchlorid, Glucose, Glycerin, Mannitol und Sorbitol.

14. Behälter enthaltend eine Zusammensetzung nach einem der Ansprüche 1 bis 13, wobei der Behälter so gestaltet ist, dass die Zusammensetzung durch Sprühen oder als Tropfen freigesetzt wird.

15. Zusammensetzung nach einem der Ansprüche 1 bis 13, zur Verwendung in therapeutischen Verfahren oder zur Verwendung in therapeutischen Verfahren für eine allergische Nasenschleimhautentzündung, Nasenverstopfung und Infektionen oder in der Behandlung von Diabetes, Migräne, Übelkeit, Raucherentwöhnung, akute Schmerzlinderung, nächtliches Einnässen, Osteoporose, Vitamin B12 Mangel oder für die Verabreichung einer intranasalen Grippeschutzimpfung.

## Revendications

1. Une composition destinée à une application sur une muqueuse comprenant de 0,1 à 10 pour cent d'un agent d'ajustement de la pression osmotique, de 0,5 à 10 pour cent d'une hydroxyalkylméthylcellulose, de 0 à 20 pour cent d'un agent physiologiquement actif, et de 0 à 30 pour cent d'un ou de plusieurs adjuvants, rapporté au poids total de la composition, le reste étant un diluant liquide, dans laquelle l'hydroxyalkylméthylcellulose a un DS allant de 1,6 à 2,7 et une SM allant de 0,40 à 1,30, dans laquelle DS est le degré de substitution de groupes méthoxyle et SM est la substitution molaire de groupes hydroxyalcoxyle, et
au moins 55 pour cent en poids du diluant liquide est de l'eau.

2. La composition de la revendication 1 dans laquelle l'agent physiologiquement actif est sélectionné parmi un ou plusieurs médicaments, un ou plusieurs agents diagnostiques, une ou plusieurs huiles essentielles, ou un ou plusieurs agents physiologiquement actifs qui sont utiles à des fins cosmétiques ou nutritionnelles.

3. La composition de la revendication 1 ou de la revendication 2 dans laquelle l'hydroxyalkylméthylcellulose a un DS allant de 1,70 à 2,20.

4. La composition de n'importe laquelle des revendications 1 à 3 dans laquelle l'hydroxyalkylméthylcellulose a une SM allant de 0,60 à 1,10.

5. La composition de n'importe laquelle des revendications 1 à 4 dans laquelle l'hydroxyalkylméthylcellulose a une viscosité allant de 20 à 50 000 mPa•s, mesurée sous la forme d'une solution à 2 % en poids dans de l'eau à 20 °C.

6. La composition de la revendication 5 dans laquelle
l'hydroxyalkylméthylcellulose a une viscosité allant de 40 à 5 000 mPa•s, mesurée sous la forme d'une solution à 2 % en poids dans de l'eau à 20 °C.

7. La composition de n'importe laquelle des revendications 1 à 6 ayant une viscosité allant de 100 à 50 000 mPa•s, mesurée à 5 °C.

8. La composition de la revendication 7 ayant une viscosité allant de 200 à 10 000 mPa•s, mesurée à 5 °C et à une vitesse de cisaillement de 10 s⁻¹.

9. La composition de n'importe laquelle des revendications 1 à 8 présentant une température de précipitation allant de 12 à 37 °C.

10. La composition de n'importe laquelle des revendications 1 à 9 destinée à une administration intranasale.

11. La composition de n'importe laquelle des revendications 1 à 10 comprenant de 0,5 à 10 pour cent de l'hydroxyalkylméthylcellulose, de 0,3 à 6,0 pour cent d'un agent d'ajustement de la pression osmotique, de 0,01 à 10 pour cent d'un agent physiologiquement actif, et de 0,01 à 20 pour cent d'un ou de plusieurs adjuvants, rapporté au poids total de la composition, le reste étant le diluant liquide.

12. La composition de la revendication 2 dans laquelle l'huile essentielle est le menthol, le salicylate de méthyle, le thymol, l'huile d'eucalyptus, le camphre, l'anis, l'orange douce, ou une combinaison de ceux-ci.

13. La composition de n'importe laquelle des revendications 1 à 12 comprenant un ou plusieurs agents d'ajustement de la pression osmotique sélectionnés parmi le chlorure de sodium, le chlorure de potassium, le dextrose, le xylitol, le chlorure de calcium, le glucose, la glycérine, le mannitol et le sorbitol.

14. Un récipient comprenant la composition de n'importe laquelle des revendications 1 à 13, le récipient étant conçu pour libérer la composition par pulvérisation ou sous la forme de gouttes.

15. La composition des revendications 1 à 13 destinée à être utilisée en thérapie ou destinée à être utilisée dans des thérapies pour la rhinite allergique, la congestion et des infections nasales ou dans le traitement du diabète, de la migraine, de la nausée, du sevrage tabagique, du soulagement de la douleur aiguë, de l'énurésie nocturne, de l'ostéoporose, de la déficience en vitamine B12 ou pour administrer un vaccin intranasal contre la grippe.
